# EUROPEAN PATENT APPLICATION

(11) **EP 4 477 664 A1**
(43) Date of publication of application: **18.12.2024**
(21) Application number: 23179262.3
(22) Date of filing: 14.06.2023
(51) Int. Cl.: C07K 14/415, C12N 15/82

(54) **COMBINING ZYP1 AND RECQ4 INHIBITION TREMENDOUSLY INCREASES CROSSOVERS IN MALE AND FEMALE MEIOSIS**

(71) Applicant: Max-Planck-Gesellschaft zur Förderung der Wissenschaften e.V., 80539 München (DE)
(72) Inventor: The designation of the inventor has not yet been filed
(74) Representative: Vossius & Partner Patentanwälte Rechtsanwälte mbB

(57) **Abstract**

The present invention relates to a method for increasing the frequency of meiotic crossover in a plant, plant part, plant tissue culture or plant cell comprising inhibiting in said plant, plant part, plant tissue culture or plant cell the expression and/or the function of the proteins ZYP1 and RECQ4. The present invention furthermore relates to a plant, plant part, plant tissue culture or plant cell, wherein the expression and/or the function of the proteins ZYP1 and RECQ4 is inhibited.

## Description

The present invention relates to a method for increasing the frequency of meiotic crossover in a plant, plant part, plant tissue culture or plant cell comprising inhibiting in said plant, plant part, plant tissue culture or plant cell the expression and/or the function of the proteins ZYP1 and RECQ4. The present invention furthermore relates to a plant, plant part, plant tissue culture or plant cell, wherein the expression and/or the function of the proteins ZYP1 and RECQ4 is inhibited.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Meiotic recombination generates crossovers (COs) between homologous chromosomes. This is initiated by the formation of numerous DNA double-strand breaks, which are repaired using the homologous chromosome as a template. A small proportion of the breaks mature into COs. Two pathways for CO formation have been described. The class I pathway, which accounts for the majority of COs in most examined species, is promoted by a group of proteins called ZMMs (Saccharomyces cerevisiae Zip1 through 4, Msh4 through 5, and Mer3) and the MutL-γ complex (MLH1/3). The presence of class I COs inhibits the formation of additional class I COs nearby along the same chromosome pair, a phenomenon known as CO interference, whose mechanism has been elusive since its first description >100 y ago. The second pathway, which accounts for a minority of COs in most species, including *Arabidopsis thaliana,* does not exhibit CO interference (1). The ZMM pathway is remarkably conserved from budding yeast to mice and A. *thaliana* with homologs of Zip2, Zip3, Zip4, Msh4, Msh5, and Mer3 being all required for class I CO formation.

Meiotic recombination promotes genetic diversity by shuffling parental chromosomes. As observed by the very first geneticists, crossovers (COs) inhibit the formation of another crossover nearby, an elusive phenomenon called crossover interference. Another intriguing observation is heterochiasmy, the marked difference in male and female crossover rates observed in many species.

Since genetic diversity is desired in particular for the generation of novel plant varieties in plant breeding programs means and methods for increasing the frequency of meiotic crossover in a plant, plant part, plant tissue or plant cell are needed. The possibility of increasing the recombination rate allows to obtain new combinations of plant characteristics, thereby facilitating the generation of plant varieties with desired properties, such as higher production yield or resistance to plant diseases or aridity.

Accordingly, the present invention relates in first aspect to a method for increasing the frequency of meiotic crossover in a plant, plant part, plant tissue culture or plant cell comprising inhibiting in said plant, plant part, plant tissue culture or plant cell the expression and/or the function of the proteins ZYP1 and RECQ4.

Meiotic crossover (also known as chromosomal crossover, crossover or crossing over) is the exchange of genetic material during sexual reproduction between two homologous chromosomes' non-sister chromatids that results in recombinant chromosomes. Crossovers are produced by meiotic recombination, which is initiated by the formation of DNA double-strand breaks and their repair using the homologous chromosome as a template. This process occurs in the context of the synaptonemal complex, a structure that holds homologous chromosomes together during the prophase of meiosis.. As discussed, meiotic crossovers are important since it promotes genetic diversity by shuffling parental chromosomes.

Crossovers correspond to points of genetic exchange, and their frequency is often measured as number per chromatid and per gamete. Genetic size in centiMorgan (cM) is also commonly used as a measure of recombination, with an average of one crossover per chromatid corresponding to 100 centiMorgan. Crossovers can also be visualized cytologically during meiosis as chiasmata, the physical point of exchange between chromosome pairs. Note that because a given chiasmata affects only two of the four chromatids of a chromosome pair, one chiasmata/crossover per chromosome pair at meiosis, corresponds to an average of 0.5 crossover per chromatid in the gamete. The calculation of crossover frequency is well established in the art and is for, example, described in reference (1). In most eukaryotes, including plants, the frequency of crossover ranges typically from 1 to 3 per chromosome pair (typical genetic size = 50-150cM); see Mercier et al. (2015), Annual Review of Plant Biology, 66:297-327.

The increase of the frequency of meiotic crossover that is achieved herein by inhibiting in said plant, plant part, plant tissue culture or plant cell the expression and/or the function of both proteins, ZYP1 and RECQ4 is higher than the frequency of meiotic crossover that is achieved herein by inhibiting in said plant, plant part, plant tissue culture or plant cell the expression and/or the function of the proteins ZYP1 or RECQ4.

The term "ZYP1 protein" corresponds to the ZYP1A and ZYP1B proteins that have two *ZYP1* genes (*ZYP1A* and *ZYP1B*), and to the ZYP1 protein in plants that have only one *ZYP1* gene. Hence, in the case of a plant, plant part, plant tissue culture or plant cell with ZYP1A and ZYP1B proteins in said plant, plant part, plant tissue culture or plant cell the expression and/or the function of the proteins ZYP1A and ZYP1B is inhibited and in in the case of a plant, plant part, plant tissue culture or plant cell with one ZYP1 protein in said plant, plant part, plant tissue culture or plant cell the expression and/or the function of this ZYP1 is inhibited.

In this respect it is noted that a ZYP1A/B tandem duplication is found in *Arabidopsis* and very close relatives (Higgins et al. (2005), Genes Dev, 19(20):2488-2500). Most diploid plant species have a single ZYP1 protein.

Similarly, the term "RECQ4 protein" corresponds to the RECQ4A and RECQ4B proteins in plants that have two RECQ4 genes (*RECQ4A* and *RECQ4B*), and to the RECQ4 protein in plants that have only one RECQ4 gene. Thus, in the case of a plant, plant part, plant tissue culture or plant cell with RECQ4A and RECQ4B proteins in said plant, plant part, plant tissue culture or plant cell the expression and/or the function of the proteins RECQ4A and RECQ4B is inhibited and in in the case of a plant, plant part, plant tissue culture or plant cell with one RECQ protein in said plant, plant part, plant tissue culture or plant cell the expression and/or the function of this RECQ4 is inhibited.

A *RECQ4A* / *RECQ4B* duplication can be found in a number of *Brassicaceae* (e.g. *Arabidopsis,* oilseed rape, cabbages) while other crops have a single RECQ4 (Séguéla-Arnaud (2015), PNAS, 112 (15):4713-4718).

For example, *Arabidopsis* have the proteins ZYP1A, ZYP1B, RECQ4A and RECQ4B while tomatoes have a single ZYP1 and a single RECQ4. It is to be understood that in the case of a plant with the proteins ZYP1A, ZYP1B, RECQ4A and RECQ4B the expression and/or the function of all four of these proteins is to be inhibited in accordance with the invention and in the case of a plant with the proteins ZYP1 and RECQ4 the expression and/or the function of both of these proteins is to be inhibited in accordance with the invention

In accordance with the present invention inhibiting the expression of the proteins ZYP1 and RECQ4 means that (i) the transcription of the genes encoding these proteins is lowered or prevented, or (ii) the translation of the mRNA encoding these proteins is lowered or prevented. In the case of (i) the transcriptional machinery and/or its interaction with the promoter of said genes and/or with expression control elements remote from the promoter such as enhancers might be interfered with. In the case of (ii) compounds interfering with the translational machinery might be interfered with. Preferably, the transcription and/or translation is reduced by at least 50%, more preferred at least 75% such as at least 90% or 95%, even more preferred at least 98% and most preferred by about 100% (e.g., as compared to the same experimental set up in the absence of the inhibition).

In accordance with the present invention inhibiting the function of the proteins ZYP1 and RECQ4 means that these proteins perform their function with lowered efficiency. The function of the protein ZYP1 (transverse filament protein) is the spatial organization of the transverse filaments of the synaptonemal complex while the function of the protein RECQ4 (RecQ Helicase 4) is its enzymatic activity as an ATP-dependent RecQ-like DNA helicase, homologue 4. Preferably, the efficiency is lowered by at least 50%, more preferred at least 75% such as at least 90% or 95%, even more preferred at least 98% and most preferred by about 100% (e.g., as compared to the same experimental set up in the absence of the inhibition).

Preferred means and methods for the inhibition will be further described herein below.

ZYP1 and RECQ4 are known to regulate class I and class II CO, respectively, predicting that the effect of inhibiting both of them should at best result in a total gain of CO that sums up the gain of CO of each individual inhibition. However and as it can be taken from the appended examples, it was surprisingly found that inhibiting both ZYP1 and RECQ4, by knocking the encoding genes out, results in an unexpected synergistic effect on deregulating meiotic recombination, both for male and female COs. In more detail, if male and female COs were combined, the number of COs by the double inhibition was 59.1 per F2 (sum of male and female gametes). The 59.1 significantly exceeds the expected number of COs when adding the gain of each individual inhibition which would only be 37.2 COs per F2. In this respect, it is also emphasized that such an unexpected synergistic effect on CO formation was not observed when combining the overexpression of HEI10 (a dose-dependent pro-class I CO factor encoding a meiotic E3 ubiquitin ligase) and the mutation of *RECQ4.*

The present invention relates in second aspect to method for preparing a plant, plant part, plant tissue culture or plant cell displaying increased frequency of meiotic crossover in plants, comprising (a) inhibiting in said plant, plant part, plant tissue or plant cell the expression and/or the function of the proteins ZYP1 and RECQ4, and (b) optionally propagating the plant, plant part, plant tissue or plant cell.

The above definitions and preferred embodiments of the first aspect of the invention apply *mutatis mutandis* to the second aspect of the invention.

Means and methods for propagating plants, plant parts, plant tissue cultures and plant cell are well-known in the art. Propagation is the process of reproducing plants from a single parent plant. There are a number of plant propagation techniques by which also plant parts can be multiplied, including division, budding, and grafting, but cutting is the most popular because it presents the lowest risk to the parent plant. The three main ways to propagate plants are cutting, division, and replanting of offsets. Taking a cutting and replanting the same creates an exact clone of the original plant. Plant tissue culture and cell culture is generally carried out under sterile growth conditions and results in the multiplication of plant cells, tissues, and organs *in vitro.* Plant cells cultured with nutrient media in an artificial environment can be clonally propagated at scale, to quickly produce plants.

In accordance with a preferred embodiment of the first and second aspect of the invention the inhibition of the proteins ZYP1 and RECQ4 is obtained by mutagenesis of the genes encoding said proteins or their promoter.

The mutagenesis of the genes encoding the ZYP1 and RECQ4 proteins or their promoter is such that the expression and/or the function of the proteins ZYP1 and RECQ4 is inhibited. A mutation in the promoter is such that the expression of the *ZYP1* and RECQ4 genes is reduced or even abolished, for example, because the binding site of transcription factors has been mutated. A mutation in the *ZYP1* and *RECQ4* genes itself may have different technical effects. Also here the expression of the *ZYP1* and *RECQ4* genes may be reduced or even abolished, for example, because the transcription start site has been mutated. The mutation in the *ZYP1* and *RECQ4* genes itself may also result in a premature stop codon or a mutated ZYP1 and RECQ4 protein with reduced or even no function. It is also possible to complete or partially delete the *ZYP1* and *RECQ4* genes or their promoters. The skilled person is aware of means and methos of effecting mutations to obtain the desired effects.

A mutation may result in the deletion, addition, and/or substitutions of nucleotides in the *ZYP1* and *RECQ4* genes and or their promoter regions and/or may result in the deletion, addition, and/or substitutions of amino acids in the ZYP1 and RECQ4 proteins.

In accordance with a more preferred embodiment of the first and second aspect the mutagenesis is the knock-out of the genes encoding ZYP1 and RECQ4 in the plant, plant part, plant tissue culture or plant cell.

In accordance with an even more preferred embodiment of the first and second aspect of the knock-out is a homozygous knock-out of the genes encoding ZYP1 and RECQ4.

A knockout, as related to genomics, refers to the use of genetic engineering to inactivate or remove one or more specific genes from an organism. Gene knockouts (also known as gene deletion or gene inactivation) are a widely used genetic engineering technique that involves the targeted removal or inactivation of a specific gene within an organism's genome.

There are two main types of gene knockouts: complete and conditional. A complete gene knockout permanently inactivates the gene, while a conditional gene knockout allows for the gene to be turned off and on at specific times or in specific tissues. Conditional knockouts are particularly useful for studying developmental processes and for understanding the role of a gene in specific cell types or tissues. The original conditional knockout method made use of a site-specific recombinase called Cre that recombines short target sequences known as LoxP. Other recombinases have since been developed and used for conditional knockout studies. Herein, a complete gene knockout is preferred.

In accordance with a more preferred embodiment of the first and second aspect, the mutagenesis is obtained by CRISPR-Cas technology, a meganuclease technology, a zinc finger nuclease technology, transcription activator-like (TAL) effector (TALE) nuclease (TALEN) technology or targeting induced local lesions (TILLING) technology.

The above techniques are in particular used in order to obtain a complete gene knockout. With this list CRISPR-Cas technology and TILLING technology are preferred while CRISPR-Cas technology is mot preferred.

CRISPR (Clustered Regularly Interspaced Short Palindromic Repeats) is a genetic engineering technique that allows for precise editing of the genome. One application of CRISPR is gene knock-out, which involves disabling or "knocking out" a specific gene in an organism. The process of gene knock-out with CRISPR involves three main steps: designing a guide RNA (gRNA) that targets a specific location in the genome, delivering the gRNA and a Cas enzyme (which acts as a molecular scissors) to the target cell, and then allowing the cell to repair the cut in the DNA. When the cell repairs the cut, it can either join the cut ends back together, resulting in a non-functional gene, or introduce a mutation that disrupts the gene's function. This technique can be used in a variety of organisms, including bacteria, yeast, plants, and animals, and it allows scientists to study the function of specific genes by observing the effects of their absence.

Meganucleases are endodeoxyribonucleases characterized by a large recognition site (double-stranded DNA sequences of 12 to 40 base pairs); as a result this site generally occurs only once in any given genome. For example, the 18-base pair sequence recognized by the I-Scel meganuclease would on average require a genome twenty times the size of the human genome to be found once by chance (although sequences with a single mismatch occur about three times per human-sized genome). Meganucleases were identified in the 1990s, and subsequent work has shown that they are particularly promising tools for genome engineering and gene editing, as they are able to efficiently induce homologous recombination, generate mutations, and alter reading frames.

Zinc finger nucleases (ZNFs) consist of DNA binding domains that can precisely target a DNA sequence. Each zinc finger can recognize codons of a desired DNA sequence, and therefore can be modularly assembled to bind to a particular sequence. These binding domains are coupled with a restriction endonuclease that can cause a double stranded break (DSB) in the DNA.[3] Repair processes may introduce mutations that destroy functionality of the gene.

TALENs contain a DNA binding domain and a nuclease that can cleave DNA. The DNA binding region consists of amino acid repeats that each recognize a single base pair of the desired targeted DNA sequence.[5] If this cleavage is targeted to a gene coding region, and non-homologous end joining (NHEJ)-mediated repair introduces insertions and deletions, a frameshift mutation often results, thus disrupting function of the gene

TILLING technology is a method in molecular biology that allows directed identification of mutations in a specific gene. TILLING has been used as a reverse genetics method in organisms such as *Arabidopsis thaliana,* zebrafish, maize, wheat, rice, soybean, tomato and lettuce. The method combines a standard and efficient technique of mutagenesis using a chemical mutagen such as ethyl methanesulfonate (EMS) with a sensitive DNA screening-technique that identifies single base mutations (also called point mutations) in a target gene. The TILLING method relies on the formation of DNA heteroduplexes that are formed when multiple alleles are amplified by PCR and are then heated and slowly cooled. A "bubble" forms at the mismatch of the two DNA strands, which is then cleaved by a single stranded nuclease. The products can then be separated by size on several different platforms.

In accordance with a preferred embodiment of the first and second aspect the inhibition of the proteins ZYP1 and RECQ4 is obtained by one or more inhibitors of the expression of the proteins ZYP1 and/or RECQ4, and/or by one or more inhibitors of the function of the proteins ZYP1 and/or RECQ4.

The inhibitor of the expression of the proteins ZYP1 and/or RECQ4 is with increasing preference a compound being capable of reducing the transcription and/or translation of the the proteins ZYP1 and/or RECQ4 by at least 50%, more preferred at least 75% such as at least 90% or 95%, even more preferred at least 98% and most preferred by about 100% (e.g., as compared to the same experimental set up in the absence of the inhibitor).

Similarly, the inhibitor of the function of the proteins ZYP1 and/or RECQ4 is with increasing preference a compound being capable of lowering the function of the proteins ZYP1 and/or RECQ4 by at least 50%, more preferred at least 75% such as at least 90% or 95%, even more preferred at least 98% and most preferred by about 100% (e.g., as compared to the same experimental set up in the absence of the inhibitor).

The efficiency of inhibition by an inhibitor can be quantified by methods comparing the level of activity in the presence of the inhibitor to that in the absence of the inhibitor. For example, the change in the amount of the nucleic acid molecule and/or the protein according to the invention formed may be used in the measurement. The efficiency of several inhibitors may be determined simultaneously in high-throughput formats. High-throughput assays, independently of being biochemical, cellular or other assays, generally may be performed in wells of microtiter plates, wherein each plate may contain 96, 384 or 1536 wells. Handling of the plates, including incubation at temperatures other than ambient temperature, and bringing into contact of test compounds with the assay mixture is preferably effected by one or more computer-controlled robotic systems including pipetting devices. In case large libraries of test compounds are to be screened and/or screening is to be effected within a short time, mixtures of, for example 10, 20, 30, 40, 50 or 100 test compounds may be added to each well. In case a well exhibits the expected activity, said mixture of test compounds may be de-convoluted to identify the one or more test compounds in said mixture giving rise to said activity.

In accordance with a more preferred embodiment of the first and second aspect the one or more inhibitors of the expression of the proteins ZYP1 and/or RECQ4 are each independently selected from a small molecule, an aptamer, a siRNA, a shRNA, a miRNA, a ribozyme, and an antisense nucleic acid molecule.

In accordance with another more preferred embodiment of the first and second aspect the one or more inhibitors of the function of the proteins ZYP1 and/or RECQ4 are selected from a small molecule, an antibody, an aptamer and an antibody mimetic, wherein the antibody mimetic is preferably selected from affibodies, adnectins, anticalins, DARPins, avimers, nanofitins, affilins, Kunitz domain peptides, Fynomers, trispecific binding molecules and probodies.

The "small molecule" as used herein is preferably an organic molecule. Organic molecules relate or belong to the class of chemical compounds having a carbon basis, the carbon atoms linked together by carbon-carbon bonds. The original definition of the term organic related to the source of chemical compounds, with organic compounds being those carbon-containing compounds obtained from plant or animal or microbial sources, whereas inorganic compounds were obtained from mineral sources. Organic compounds can be natural or synthetic. The organic molecule is preferably an aromatic molecule and more preferably a heteroaromatic molecule. In organic chemistry, the term aromaticity is used to describe a cyclic (ring-shaped), planar (flat) molecule with a ring of resonance bonds that exhibits more stability than other geometric or connective arrangements with the same set of atoms. Aromatic molecules are very stable, and do not break apart easily to react with other substances. In a heteroaromatic molecule at least one of the atoms in the aromatic ring is an atom other than carbon, e.g. N, S, or O. For all above-described organic molecules the molecular weight is preferably in the range of 200 Da to 1500 Da and more preferably in the range of 300 Da to 1000 Da.

Alternatively, the "small molecule" in accordance with the present invention may be an inorganic compound. Inorganic compounds are derived from mineral sources and include all compounds without carbon atoms (except carbon dioxide, carbon monoxide and carbonates). Preferably, the small molecule has a molecular weight of less than about 2000 Da, or less than about 1000 Da such as less than about 500 Da, and even more preferably less than about Da amu. The size of a small molecule can be determined by methods well-known in the art, e.g., mass spectrometry. The small molecules may be designed, for example, based on the crystal structure of the target molecule, where sites presumably responsible for the biological activity can be identified and verified in in vivo assays such as in vivo high-throughput screening (HTS) assays.

In accordance with the present invention, the term "small interfering RNA (siRNA)", also known as short interfering RNA or silencing RNA, refers to a class of 18 to 30, preferably 19 to 25, most preferred 21 to 23 or even more preferably 21 nucleotide-long double-stranded RNA molecules that play a variety of roles in biology. Most notably, siRNA is involved in the RNA interference (RNAi) pathway where the siRNA interferes with the expression of a specific gene. In addition to their role in the RNAi pathway, siRNAs also act in RNAi-related pathways, e.g. as an antiviral mechanism or in shaping the chromatin structure of a genome.

siRNAs naturally found in nature have a well defined structure: a short double-strand of RNA (dsRNA) with 2-nt 3' overhangs on either end. Each strand has a 5' phosphate group and a 3' hydroxyl (-OH) group. This structure is the result of processing by dicer, an enzyme that converts either long dsRNAs or small hairpin RNAs into siRNAs. siRNAs can also be exogenously (artificially) introduced into cells to bring about the specific knockdown of a gene of interest. Essentially any gene for which the sequence is known can thus be targeted based on sequence complementarity with an appropriately tailored siRNA. The double-stranded RNA molecule or a metabolic processing product thereof is capable of mediating target-specific nucleic acid modifications, particularly RNA interference and/or DNA methylation. Exogenously introduced siRNAs may be devoid of overhangs at their 3' and 5' ends, however, it is preferred that at least one RNA strand has a 5'- and/or 3'-overhang. Preferably, one end of the double-strand has a 3'-overhang from 1 to 5 nucleotides, more preferably from 1 to 3 nucleotides and most preferably 2 nucleotides. The other end may be blunt-ended or has up to 6 nucleotides 3'-overhang. In general, any RNA molecule suitable to act as siRNA is envisioned in the present invention. The most efficient silencing was so far obtained with siRNA duplexes composed of 21-nt sense and 21-nt antisense strands, paired in a manner to have a 2-nt 3'- overhang. The sequence of the 2-nt 3' overhang makes a small contribution to the specificity of target recognition restricted to the unpaired nucleotide adjacent to the first base pair (Elbashir et al. 2001). 2'-deoxynucleotides in the 3' overhangs are as efficient as ribonucleotides, but are often cheaper to synthesize and probably more nuclease resistant.

A short hairpin RNA (shRNA) is a sequence of RNA that makes a tight hairpin turn that can be used to silence gene expression via RNA interference. shRNA uses a vector introduced into cells and utilizes the U6 promoter to ensure that the shRNA is always expressed. This vector is usually passed on to daughter cells, allowing the gene silencing to be inherited. The shRNA hairpin structure is cleaved by the cellular machinery into siRNA, which is then bound to the RNA-induced silencing complex (RISC). This complex binds to and cleaves mRNAs which match the siRNA that is bound to it. si/shRNAs to be used in the present invention are preferably chemically synthesized using appropriately protected ribonucleoside phosphoramidites and a conventional DNA/RNA synthesizer. Suppliers of RNA synthesis reagents are Proligo (Hamburg, Germany), Dharmacon Research (Lafayette, CO, USA), Pierce Chemical (part of Perbio Science, Rockford, IL, USA), Glen Research (Sterling, VA, USA), ChemGenes (Ashland, MA, USA), and Cruachem (Glasgow, UK). Most conveniently, siRNAs or shRNAs are obtained from commercial RNA oligo synthesis suppliers, which sell RNA-synthesis products of different quality and costs. In general, the RNAs applicable in the present invention are conventionally synthesized and are readily provided in a quality suitable for RNAi.

Further molecules effecting RNAi include, for example, microRNAs (miRNA). Said RNA species are single-stranded RNA molecules. Endogenously present miRNA molecules regulate gene expression by binding to a complementary mRNA transcript and triggering of the degradation of said mRNA transcript through a process similar to RNA interference. Accordingly, exogenous miRNA may be employed as an inhibitor of ZYP1 or RECQ4 after introduction into the respective cells.

The term "antisense nucleic acid molecule", as used herein, refers to a nucleic acid which is complementary to a target nucleic acid. An antisense molecule in accordance with the invention is capable of interacting with the target nucleic acid, more specifically it is capable of hybridizing with the target nucleic acid. Due to the formation of the hybrid, transcription of the target gene(s) and/or translation of the target mRNA is reduced or blocked. Standard methods relating to antisense technology have been described (see, e.g., Melani et al., Cancer Res. (1991) 51 :2897-2901).

The term "antibody" as used in accordance with the present invention comprises, for example, polyclonal or monoclonal antibodies. Furthermore, also derivatives or fragments thereof, which still retain the binding specificity to the target, e.g. ZYP1 or RECQ4, are comprised in the term "antibody". Antibody fragments or derivatives comprise, inter alia, Fab or Fab' fragments, Fd, F(ab')2, Fv or scFv fragments, single domain VH or V-like domains, such as VhH or V-NAR-domains, as well as multimeric formats such as minibodies, diabodies, tribodies or triplebodies, tetrabodies or chemically conjugated Fab'-multimers (see, for example, Harlow and Lane "Antibodies, A Laboratory Manual", Cold Spring Harbor Laboratory Press, 198; Harlow and Lane "Using Antibodies: A Laboratory Manual" Cold Spring Harbor Laboratory Press, 1999; Altshuler EP, Serebryanaya DV, Katrukha AG. 2010, Biochemistry (Mosc)., vol. 75(13), 1584; Holliger P, Hudson PJ. 2005, Nat Biotechnol., vol. 23(9), 1126). The multimeric formats in particular comprise bispecific antibodies that can simultaneously bind to two different types of antigen. The first antigen can be found on ZYP1 and the second on RECQ4. Non-limting examples of bispecific antibodies formats are Biclonics (bispecific, full length human IgG antibodies), DART (Dual-affinity Retargeting Antibody) and BiTE (consisting of two single-chain variable fragments (scFvs) of different antibodies) molecules (Kontermann and Brinkmann (2015), Drug Discovery Today, 20(7):838-847).

The term "antibody" also includes embodiments such as chimeric (human constant domain, non-human variable domain), single chain and humanised (human antibody with the exception of non-human CDRs) antibodies.

Various techniques for the production of antibodies are well known in the art and described, e.g. in Harlow and Lane (1988) and (1999) and Altshuler et al., 2010, loc. cit. Thus, polyclonal antibodies can be obtained from the blood of an animal following immunisation with an antigen in mixture with additives and adjuvants and monoclonal antibodies can be produced by any technique which provides antibodies produced by continuous cell line cultures. Examples for such techniques are described, e.g. in Harlow E and Lane D, Cold Spring Harbor Laboratory Press, 1988; Harlow E and Lane D, Using Antibodies: A Laboratory Manual, Cold Spring Harbor Laboratory Press, 1999 and include the hybridoma technique originally described by Köhler and Milstein, 1975, the trioma technique, the human B-cell hybridoma technique (see e.g. Kozbor D, 1983, Immunology Today, vol.4, 7; Li J, et al. 2006, PNAS, vol. 103(10), 3557) and the EBV-hybridoma technique to produce human monoclonal antibodies (Cole et al., 1985, Alan R. Liss, Inc, 77-96). Furthermore, recombinant antibodies may be obtained from monoclonal antibodies or can be prepared de novo using various display methods such as phage, ribosomal, mRNA, or cell display. A suitable system for the expression of the recombinant (humanised) antibodies may be selected from, for example, bacteria, yeast, insects, mammalian cell lines or transgenicanimals or plants (see, e.g., US patent 6,080,560; Holliger P, Hudson PJ. 2005, Nat Biotechnol., vol. 23(9), 11265). Further, techniques described for the production of single chain antibodies (see, inter alia, US Patent 4,946,778) can be adapted to produce single chain antibodies specific for an epitope of ZYP1 or RECQ4. Surface plasmon resonance as employed in the BIAcore system can be used to increase the efficiency of phage antibodies.

As used herein, the term "antibody mimetics" refers to compounds which, like antibodies, can specifically bind antigens, such as ZYP1 and/or RECQ4 in the present case, but which are not structurally related to antibodies. Antibody mimetics are usually artificial peptides or proteins with a molar mass of about 3 to 20 kDa. For example, an antibody mimetic may be selected from the group consisting of affibodies, adnectins, anticalins, DARPins, avimers, nanofitins, affilins, Kunitz domain peptides, Fynomers^{®}, trispecific binding molecules and probodies. These polypeptides are well known in the art and are described in further detail herein below.

The term "affibody", as used herein, refers to a family of antibody mimetics which is derived from the Z-domain of staphylococcal protein A. Structurally, affibody molecules are based on a three-helix bundle domain which can also be incorporated into fusion proteins. In itself, an affibody has a molecular mass of around 6kDa and is stable at high temperatures and under acidic or alkaline conditions. Target specificity is obtained by randomisation of 13 amino acids located in two alpha-helices involved in the binding activity of the parent protein domain (Feldwisch J, Tolmachev V.; (2012) Methods Mol Biol. 899:103-26).

The term "adnectin" (also referred to as "monobody"), as used herein, relates to a molecule based on the 10th extracellular domain of human fibronectin III (10Fn3), which adopts an Ig-like β-sandwich fold of 94 residues with 2 to 3 exposed loops, but lacks the central disulphide bridge (Gebauer and Skerra (2009) Curr Opinion in Chemical Biology 13:245-255). Adnectins with the desired target specificity, i.e. against ZYP1 and/or RECQ4, can be genetically engineered by introducing modifications in specific loops of the protein.

The term "anticalin", as used herein, refers to an engineered protein derived from a lipocalin (Beste G, Schmidt FS, Stibora T, Skerra A. (1999) Proc Natl Acad Sci USA. 96(5):1898-903; Gebauer and Skerra (2009) Curr Opinion in Chemical Biology 13:245-255). Anticalins possess an eight-stranded β-barrel which forms a highly conserved core unit among the lipocalins and naturally forms binding sites for ligands by means of four structurally variable loops at the open end. Anticalins, although not homologous to the IgG superfamily, show features that so far have been considered typical for the binding sites of antibodies: (i) high structural plasticity as a consequence of sequence variation and (ii) elevated conformational flexibility, allowing induced fit to targets with differing shape.

As used herein, the term "DARPin" refers to a designed ankyrin repeat domain (166 residues), which provides a rigid interface arising from typically three repeated β-turns. DARPins usually carry three repeats corresponding to an artificial consensus sequence, wherein six positions per repeat are randomised. Consequently, DARPins lack structural flexibility (Gebauer and Skerra, 2009).

The term "avimer", as used herein, refers to a class of antibody mimetics which consist of two or more peptide sequences of 30 to 35 amino acids each, which are derived from A-domains of various membrane receptors and which are connected by linker peptides. Binding of target molecules occurs via the A-domain and domains with the desired binding specificity, i.e. for ZYP1 or RECQ4, can be selected, for example, by phage display techniques. The binding specificity of the different A-domains contained in an avimer may, but does not have to be identical (Weidle UH, et al., (2013), Cancer Genomics Proteomics; 10(4):155-68).

A "nanofitin" (also known as affitin) is an antibody mimetic protein that is derived from the DNA binding protein Sac7d of Sulfolobus acidocaldarius. Nanofitins usually have a molecular weight of around 7kDa and are designed to specifically bind a target molecule, such as e.g. ZYP1 or RECQ4, by randomising the amino acids on the binding surface (Mouratou B, Béhar G, Paillard-Laurance L, Colinet S, Pecorari F., (2012) Methods Mol Biol.; 805:315-31).

The term "affilin", as used herein, refers to antibody mimetics that are developed by using either gamma-B crystalline or ubiquitin as a scaffold and modifying amino-acids on the surface of these proteins by random mutagenesis. Selection of affilins with the desired target specificity, i.e. against ZYP1 or RECQ4, is effected, for example, by phage display or ribosome display techniques. Depending on the scaffold, affilins have a molecular weight of approximately 10 or 20kDa. As used herein, the term affilin also refers to di- or multimerised forms of affilins (Weidle UH, et al., (2013), Cancer Genomics Proteomics; 10(4):155-68).

A "Kunitz domain peptide" is derived from the Kunitz domain of a Kunitz-type protease inhibitor such as bovine pancreatic trypsin inhibitor (BPTI), amyloid precursor protein (APP) or tissue factor pathway inhibitor (TFPI). Kunitz domains have a molecular weight of approximately 6kDA and domains with the required target specificity, i.e. against ZYP1 or RECQ4, can be selected by display techniques such as phage display (Weidle et al., (2013), Cancer Genomics Proteomics; 10(4):155-68).

As used herein, the term "Fynomer" refers to a non-immunoglobulin-derived binding polypeptide derived from the human Fyn SH3 domain. Fyn SH3-derived polypeptides are well-known in the art and have been described e.g. in Grabulovski et al. (2007) JBC, 282, p. 3196-3204, WO 2008/022759, Bertschinger et al (2007) Protein Eng Des Sel 20(2):57-68, Gebauer and Skerra (2009) Curr Opinion in Chemical Biology 13:245-255, or Schlatter et al. (2012), MAbs 4:4, 1-12).

The term "trispecific binding molecule" as used herein refers to a polypeptide molecule that possesses three binding domains and is thus capable of binding, preferably specifically binding to three different epitopes. At least one of these three epitopes is an epitope of the protein of the fourth aspect of the invention. The two other epitopes may also be epitopes of the protein of the fourth aspect of the invention or may be epitopes of one or two different antigens. The trispecific binding molecule is preferably a TriTac. A TriTac is a T-cell engager which comprised of three binding domains being designed to have an extended serum half-life and be about one-third the size of a monoclonal antibody.

The term "evibodies" refers to specific binding proteins based on soluble CTLA-4 variable domain as a scaffold (Nuttall et al, Proteins, 36:217-227 (1999))

As used herein, the term "probody" refers to a protease-activatable antibody prodrug. A probody consists of an authentic IgG heavy chain and a modified light chain.

Aptamers are nucleic acid molecules or peptide molecules that bind a specific target molecule. Aptamers are usually created by selecting them from a large random sequence pool, but natural aptamers also exist in riboswitches. Aptamers can be combined with ribozymes to self-cleave in the presence of their target molecule. These compound molecules have additional research, industrial and clinical applications (Osborne et. al. (1997), Current Opinion in Chemical Biology, 1 :5-9; Stull & Szoka (1995), Pharmaceutical Research, 12, 4:465-483).

Nucleic acid aptamers are nucleic acid species that normally consist of (usually short) strands of oligonucleotides. Typically, they have been engineered through repeated rounds of in vitro selection or equivalently, SELEX (systematic evolution of ligands by exponential enrichment) to bind to various molecular targets such as small molecules, proteins, nucleic acids, and even cells, tissues and organisms.

Peptide aptamers are usually peptides or proteins that are designed to interfere with other protein interactions inside cells. They consist of a variable peptide loop attached at both ends to a protein scaffold. This double structural constraint greatly increases the binding affinity of the peptide aptamer to levels comparable to an antibody's (nanomolar range). The variable peptide loop typically comprises 10 to 20 amino acids, and the scaffold may be any protein having good solubility properties. Currently, the bacterial protein Thioredoxin-A is the most commonly used scaffold protein. Peptide aptamer selection can be made using different systems, but the most widely used is currently the yeast two-hybrid system.

Aptamers offer the utility for biotechnological and therapeutic applications as they offer molecular recognition properties that rival those of the commonly used biomolecules, in particular antibodies. In addition to their discriminatory recognition, aptamers offer advantages over antibodies as they can be engineered completely in a test tube, are readily produced by chemical synthesis, possess desirable storage properties, and elicit little or no immunogenicity in therapeutic applications. Non-modified aptamers are cleared rapidly from the bloodstream, with a half-life of minutes to hours, mainly due to nuclease degradation and clearance from the body by the kidneys, a result of the aptamers' inherently low molecular weight. Unmodified aptamer applications currently focus on treating transient conditions such as blood clotting, or treating organs such as the eye where local delivery is possible. This rapid clearance can be an advantage in applications such as in vivo diagnostic imaging. Several modifications, such as 2'-fluorine-substituted pyrimidines, polyethylene glycol (PEG) linkage, fusion to albumin or other half-life extending proteins etc. are available to scientists such that the half-life of aptamers can be increased for several days or even weeks.

A ribozyme (from ribonucleic acid enzyme, also called RNA enzyme or catalytic RNA) is an RNA molecule that catalyses a chemical reaction. Many natural ribozymes catalyse either their own cleavage or the cleavage of other RNAs, but they have also been found to catalyse the aminotransferase activity of the ribosome. Non-limiting examples of well-characterised small self-cleaving RNAs are the hammerhead, hairpin, hepatitis delta virus, and *in* vitro-selected lead-dependent ribozymes, whereas the group I intron is an example for larger ribozymes. The principle of catalytic self-cleavage has become well established in recent years. The hammerhead ribozymes are characterised best among the RNA molecules with ribozyme activity. Since it was shown that hammerhead structures can be integrated into heterologous RNA sequences and that ribozyme activity can thereby be transferred to these molecules, it appears that catalytic antisense sequences for almost any target sequence can be created, provided the target sequence contains a potential matching cleavage site. The basic principle of constructing hammerhead ribozymes is as follows: A region of interest of the RNA, which contains the GUC (or CUC) triplet, is selected. Two oligonucleotide strands, each usually with 6 to 8 nucleotides, are taken and the catalytic hammerhead sequence is inserted between them. The best results are usually obtained with short ribozymes and target sequences.

A recent development, also useful in accordance with the present invention, is the combination of an aptamer, recognizing a small compound, with a hammerhead ribozyme. The conformational change induced in the aptamer upon binding the target molecule can regulate the catalytic function of the ribozyme.

The present invention relates in second aspect to a plant, plant part, plant tissue culture or plant cell, wherein the expression and/or the function of the proteins ZYP1 and RECQ4 is inhibited.

The above definitions and preferred embodiments of the first and second aspect of the invention apply *mutatis mutandis* to the third aspect of the invention.

This means, for example, that also in connection with the third aspect the expression and/or the function of the proteins ZYP1 and RECQ4 can be inhibited by any of the means and methods as described herein above in connection with the first and second aspect, such as a small molecule, an aptamer, a siRNA, a shRNA, a miRNA, a ribozyme, an antisense nucleic acid molecule, an antibody, an aptamer or an antibody mimetic.

In accordance with a preferred embodiment of the third aspect the genes encoding ZYP1 and RECQ4 are mutated, are preferably knocked-out and are most preferably homozygously knocked-out.

As discussed above, a homozygous knocked-out of the genes encoding ZYP1 and RECQ4 not only inhibits but completely abolishes the expression and activity of ZYP1 and RECQ4 in the plant, plant part, plant tissue culture or plant cell and a homozygous knocked-out is therefore most preferred and illustrated by the appended examples.

In accordance with a preferred embodiment of the first, second and third aspect
the ZYP1 protein (a) comprises or consists of an amino acid sequence being with increasing preference at least 50%, at least 60%, at least 70%, at least 80%, at least 90%, and at least 95% to SEQ ID NO: 1 or is encoded by a nucleotide sequence being with increasing preference at least 50%, at least 60%, at least 70%, at least 80%, at least 90%, and at least 95% to SEQ ID NO: 2; and/or (b) comprises or consists of an amino acid sequence being with increasing preference at least 50%, at least 60%, at least 70%, at least 80%, at least 90%, and at least 95% to SEQ ID NO: 3 or is encoded by a nucleotide sequence being with increasing preference at least 50%, at least 60%, at least 70%, at least 80%, at least 90%, and at least 95% to SEQ ID NO: 4; and/or
the RECQ4 protein (c) comprises or consists of an amino acid sequence being with increasing preference at least 50%, at least 60%, at least 70%, at least 80%, at least 90%, and at least 95% to SEQ ID NO: 5 or is encoded by a nucleotide sequence being with increasing preference at least 50%, at least 60%, at least 70%, at least 80%, at least 90%, and at least 95% to SEQ ID NO: 6, and/or (d) comprises or consists of an amino acid sequence being with increasing preference at least 50%, at least 60%, at least 70%, at least 80%, at least 90%, and at least 95% to SEQ ID NO: 7 or is encoded by a nucleotide sequence being with increasing preference at least 50%, at least 60%, at least 70%, at least 80%, at least 90%, and at least 95% to SEQ ID NO: 8.

In the appended examples the claimed subject-matter is illustrated based on an *Arabidopsis* plant wherein the genes encoding ZYP1 and RECQ4 are knocked out. As discussed herein above, in *Arabidopsis* the genes encoding ZYP1 and RECQ4 are both duplicated, so that *Arabidopsis* has the proteins ZYP1A, ZYP1B, RECQ4A and RECQ4B. The *Arabidopsis* plant in the appended examples is therefore a *zyp1a*^{*-*/}*⁻, zyp1b*^{*-*/}*⁻, recq4a*^{*-*/*-*} and *recq4b*^{*-*/*-*} plant.

SEQ ID NOs 1 and 2 are the amino acid and nucleotide sequence of ZYP1A of A. *thaliana.* SEQ ID NOs 3 and 4 are the amino acid and nucleotide sequence of ZYP1 B of A. *thaliana.* SEQ ID NOs 5 and 6 are the amino acid and nucleotide sequence of RECQ4A of A. *thaliana.* SEQ ID NOs 7 and 8 are the amino acid and nucleotide sequence of RECQ4B of A. *thaliana.*

In accordance with another preferred embodiment of the first, second and third aspect the plant, plant part, plant tissue culture or plant cell is derived from a plant being selected from *Arabidopsis* (e.g. A. *thaliana),* potato, cucumber, watermelon, sweet potato, eggplant, lettuce, cow pea, cassava, peanut, cotton, chick pea, onion, apple, corn, rice, wheat, barley, sorghum, millet oats, rye, triticale, cereals, soybean, alfalfa, leguminous crops, sugar beet, fodder beet, papaya, banana, plantains, fruits, grapevines, nuts, oilseed rape, sunflower, oil crops, squash cucumber, melons, cucurbits, cotton, fiber plants, sugarcane, palm, jatropha, fuel crops, cabbages, tomato, pepper, vegetables, ornamentals, shrubs, poplar, eucalyptus, trees, evergreens, grasses, coffee plants, tea plants, tobacco plants, hop plants, and rubber.

While in the appended examples the claimed subject-matter is illustrated based on the model plant *Arabidopsis* the proteins ZYP1 and RECQ4 as well as the genes encoding ZYP1 and RECQ4 can be found throughout the plant kingdom. RECQ4 is even evolutionary conserved in yeast. In addition, the genome organization or even the entire genome sequences are well known for most plants that are of commercial interest (e.g. crop plants, animal feed plants, medical plants, fruits and legumes) and if not the genome organization of plant can be determined by routine means. It is there also known or can be found out by routine means whether a plant has single *ZYP1* and/or *RECQ4* genes or has duplicated *ZYP1* and/or *RECQ4* genes. For this reason the inhibition of proteins ZYP1 and RECQ4 is not limited to any particular kind of plant, such as an *Arabidopsis* but can be executed in essentially all plants. The above listed plants are particularly preferred because they are of commercial interest and/or model plants often being used in plant science.

In accordance with a further preferred embodiment of the first, second and third aspect (a) the plant part is selected from seeds, leaves, flowers, anthers, ovules, fruits, stem cultures, rhizomes, tubers and bulbs, and is preferably flowers anthers, or ovules; (b) the plant tissue culture is selected from flower tissue, anthers tissue, ovules tissue, meristematic tissue, protective tissue, parenchyma, sclerenchyma, collenchyma, xylem and phloem, and is preferably flower tissue, anthers tissue or ovules tissue; and/or (c) the plant cell is from callus tissue, plant embryos, meristematic regions, reproductive tissues, meiocytes, gametophytes, sporophytes, pollen, egg, macrospore, or microspores and is preferably from meiocytes.

While the nature of the plant part, plant tissue culture or plant cell is not particularly limited the above kinds of plant parts, plant tissue cultures or plant cells are preferred.

The plant part is preferably flowers, anthers or ovules. Anthers and ovules are part of the flower. The anthers are the part of the stamen where pollen is produced and ovules can be found in the pistil. In angiosperms, meiosis takes place in sporogenous cells that develop *de novo* from somatic cells in anthers or ovules. Hence, in plants meiotic CO generally takes place in anthers or ovules. Accordingly, also the plant tissue culture is preferably flower tissue, anthers tissue or ovules tissue and the plant cells are preferably meiocytes. A meiocyte is a type of cell that differentiates into a gamete through the process of meiosis. Through meiosis, the diploid meiocyte divides into four genetically different haploid gametes. Meiocytes can be found in the anthers and ovules.

Regarding the embodiments characterized in this specification, in particular in the claims, it is intended that each embodiment mentioned in a dependent claim is combined with each embodiment of each claim (independent or dependent) said dependent claim depends from. For example, in case of an independent claim 1 reciting 3 alternatives A, B and C, a dependent claim 2 reciting 3 alternatives D, E and F and a claim 3 depending from claims 1 and 2 and reciting 3 alternatives G, H and I, it is to be understood that the specification unambiguously discloses embodiments corresponding to combinations A, D, G; A, D, H; A, D, I; A, E, G; A, E, H; A, E, I; A, F, G; A, F, H; A, F, I; B, D, G; B, D, H; B, D, I; B, E, G; B, E, H; B, E, I; B, F, G; B, F, H; B, F, I; C, D, G; C, D, H; C, D, I; C, E, G; C, E, H; C, E, I; C, F, G; C, F, H; C, F, I, unless specifically mentioned otherwise.

Similarly, and also in those cases where independent and/or dependent claims do not recite alternatives, it is understood that if dependent claims refer back to a plurality of preceding claims, any combination of subject-matter covered thereby is considered to be explicitly disclosed. For example, in case of an independent claim 1, a dependent claim 2 referring back to claim 1, and a dependent claim 3 referring back to both claims 2 and 1, it follows that the combination of the subject-matter of claims 3 and 1 is clearly and unambiguously disclosed as is the combination of the subject-matter of claims 3, 2 and 1. In case a further dependent claim 4 is present which refers to any one of claims 1 to 3, it follows that the combination of the subject-matter of claims 4 and 1, of claims 4, 2 and 1, of claims 4, 3 and 1, as well as of claims 4, 3, 2 and 1 is clearly and unambiguously disclosed.

The above considerations apply *mutatis mutandis* to all appended claims.

The figures show:

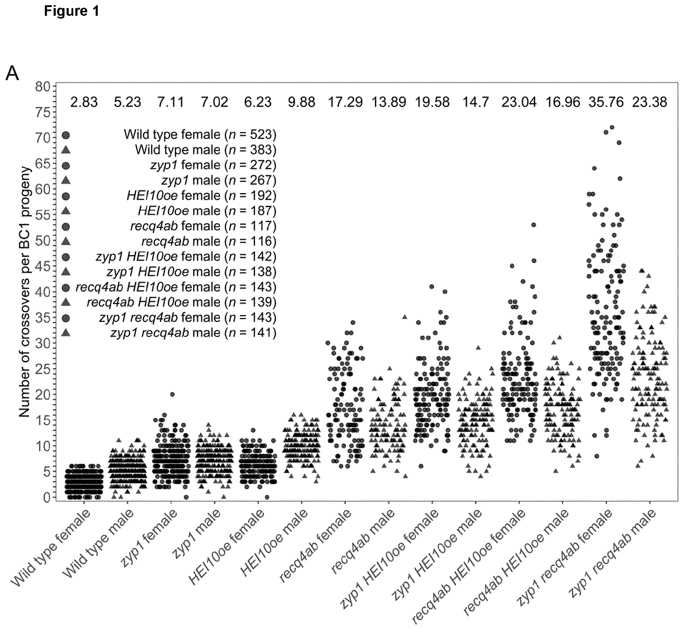

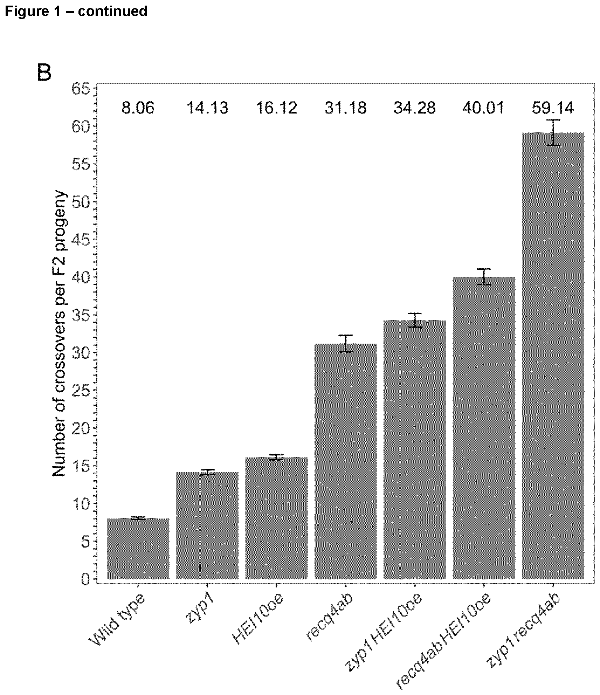

**Figure 1** **- Combination of *zyp1* and *recq4* mutations massively increases COs.** (A) The number of crossovers per gamete in male and female BC1 populations of wild type, *zyp1, HEI10oe, recq4ab and* combinations. Each dot represents a gamete, and the mean crossover number per gamete is shown on the top. (B) The number of crossovers per progeny in pseudo-F2 populations (sum of male and female gametes, mean +/- SEM), of wild type, *zyp1, HEI10oe, recq4ab, zyp1 HEI10oe, recq4ab HEI10oe* and *zyp1 recq4ab.* The mean crossover numbers is shown on the top.

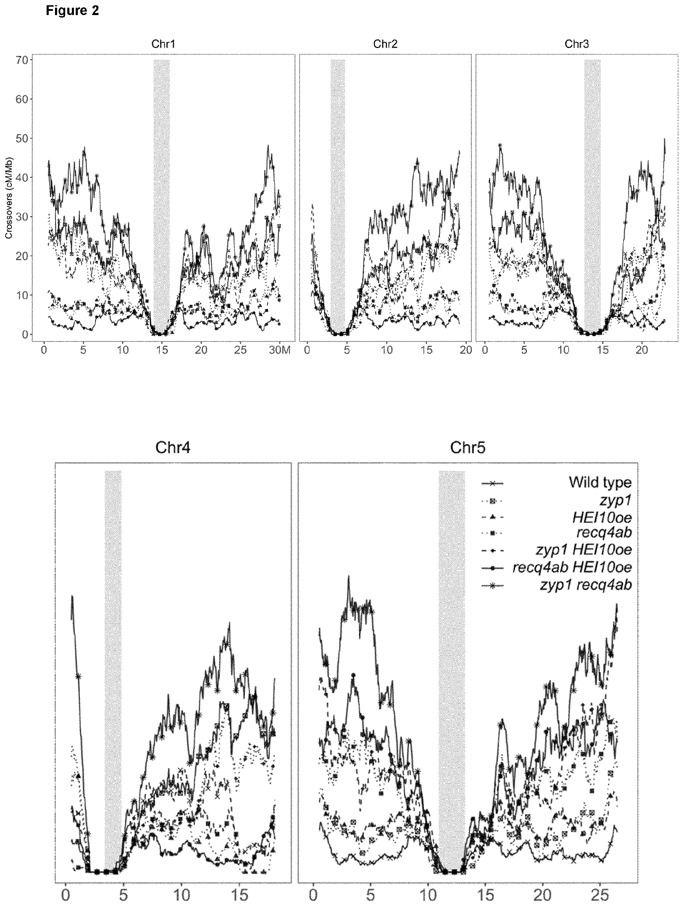

**Figure 2** - **The distribution of crossovers along the five chromosomes.** The centromere regions are indicated by a grey shading. The crossover frequency in cM/Mb is the average of male and female gametes. The analysis is done with 1-Mb windows and 50-Kb sliding steps.

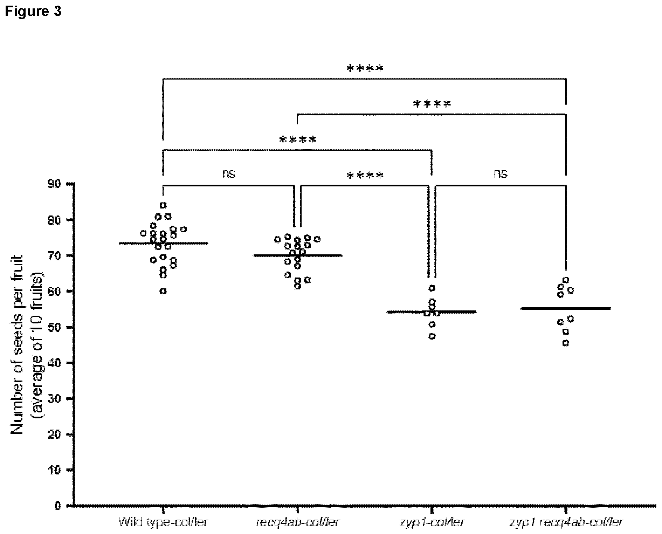

**Figure 3** - **Analysis of fertility in wild type, *zyp1, recq4ab* and *zyp1 recq4ab* mutants.** Each dot represents the fertility of an individual plant, measured as the number of seeds per fruit averaged on 10 fruits. The horizontal bar shows the mean for a given genotype. The tests are one-way ANOVA followed by Fisher's least significant difference (LSD).

The Examples illustrate the invention:

### Example 1 - Combining zyp1 and recq4 tremendously increases crossovers in both male and female Meiosis.

To assess the effect on meiotic recombination of the simultaneous depletion of *ZYP1* and *RECQ4,* crossovers (COs) frequencywas genetically measured in hybrid plants, using the genetic polymorphisms between the Col and Ler accessions. Thus, Col/Ler F1 hybrid plants were generated that were either wild type, mutant for *ZYP1,* mutant for *RECQ4A*/*B,* or mutant for both *ZYP1* and *RECQ4A*/*B.* To measure separately male and female recombination, these F1 hybrid plants were backcrossed as male and female with wild type Col plants. The obtained male and female BC1 populations were sequenced with Illumina short reads and COs were called as previously described^{1 2}. In the wild type, the average COs number per transmitted gamete was higher in male than female meiosis (Fig. 1A), consistent with previous results^{1,3}. In *zyp1*²*,* the CO number was increased compared to wild type, reaching 7.1 (2.5-fold/wild type) in females and 7.0 (1.3-fold/wild type) in males, in accordance with previous reports.

Overexpression of HEI10 *(HEI1Ooe)^{4,5}* roughly doubled the number of COs in both males and females, reaching 9.9 and 6.2, respectively. A combination of *zyp1* mutation and HEI10 overexpression (*HE110oe*) had a cumulative effect on class I CO, as previously shown⁶. In *recq4ab,* the average number of COs per gamete was increased to high levels, reaching 17.3 (6.1-fold/wild type) in females and 13.9 (2.6-fold/wild type) in males (Fig. 1A), confirming the major role of RECQ4A/B in regulating meiotic COS^{7,8}. As shown before, the overexpression of HEI10 in the *recq4* context slightly increased CO levels⁸. Strikingly, it was observed that in *zyp1 recq4ab,* the COs number per progeny was tremendously increased to 35.8 in females (12.7-fold/wild type) and 23.4 in males (4.4-fold/wild type).

If male and female COs were combined, simulating selfing (Fig.1B), the number of COs in *zyp1 recq4ab* is 59.1 COs per F2, which is ~7 times of wild type, ~4 times of *zyp1,* -twice of *recq4ab* (Fig. 1B). ZYP1 and RECQ4 are known to regulate class I and class II COs, respectively, predicting that their effect on the total number of COs should be additive. Under the hypothesis of additivity, the expected level of COs in *zyp1 recq4ab* can be calculated by adding to the *recq4ab* CO counts (31.2), the gain of Class I COs caused by the *zyp1* single mutation (14.1-8.1=6) which predicts 37.2 COs per F2. The observed COs frequency in *zyp1 recq4ab* (59.1), is much higher than this prediction. Thus, the massive increase of COs in *zyp1 recq4ab* is the consequence of an unexpected synergistic effect on deregulating meiotic recombination.

Similar to *zyp1,* overexpression of the pro-class I factor HEI10 also leads to an increase in class I COs⁹, with a slightly larger effect on CO counts than *zyp1* (Fig. 1B). Previous studies showed that overexpressing HEI10 in *recq4ab* further increases CO frequency⁴. However, in contrast to *zyp1,* HEI0oe leads to only a limited increase when combined with *recq4ab* (40.0 COs per F2 in *recq4ab HEI10oe,* vs 31.2 in *recq4ab,* Fig. 1B*)*. Following the same logic as above, the additivity of the *recq4ab* and *HEI10oe* effects would predict 37.2 COs in *recq4ab HEI10oe* (31.2+8.1=39.2), which is very close to the observed value (40.0). Thus, the synergetic effect on crossover formation observed when combining *zyp1* and *recq4ab* is not observed when combining *recq4ab* and *HEI10oe.*

The extremely high recombination levels as observed in *zyp1 recq4ab* are unprecedented in any previously described hyper-recombinant mutants^{4-6,10,11}.

In addition, analysis of CO distribution along the genome, showed that *zyp1 recq4ab* has the highest CO level at any position along the arm of the five chromosomes, compared with wild type, *zyp1, HEI10oe, recq4ab, zyp1 HEI10oe and recq4ab HEI10oe* (Fig. 2). However, in all genetic backgrounds, CO were absent in peri-centromeric regions, suggesting that the COs are suppressed by other mechanisms in these regions.

Fertility was measured by counting the number of seeds per fruit. *recq4ab* mutant showed no reduction in fertility compared with wild type, consistent with previous results⁴ (p=0.2361, Fig. 3). In *zyp1* and *zyp1 recq4ab,* fertility is slightly but significantly reduced compared with wild type (p<0.0001, Fig. 3). Interestingly, *zyp1 recq4ab* showed a level of fertility similar to *zyp1* (p=0.9833, Fig. 3), despite a dramatic increase in CO frequency. Although elevated levels of meiotic crossover associates with a slight reduction in fertility, the resulting fertility (>75% of wild type), making possible the use of elevated recombination in plant breeding programs.

### Example 2 - Methods

### Plant materials and growth conditions

*Arabidopsis thaliana* plants were cultivated in Polyklima growth chambers (16-h day, 21.5 °C, 280 µM; 8-h night, 18 °C: 60% humidity). Wild type Col-0 and L*er*-1 are 186AV1B4 and 213AV1B1 from the Versailles stock center (http://publiclines.versailles.inra.fr/). The *zyp1-1* (Col), *zyp1-6* (*Ler*), *recq4a-4* (Col, N419423), *recq4b-2* (Col, N511130), *recq4a-W387X (Ler),* were characterized previously ^{10,11}. Genotyping of the mutants was carried out by PCR amplification (Supplementary Data 1). To generate the triple heterozygous *zyp1-1*^{+/}*⁻ recq4a*^{+/}*⁻ recq4b*^{+/*-*} in Col, *zyp1-1*^{+/*-*} plants were crossed with *recq4a*^{+/*-*} plants to get *zyp1-1*^{+/}*⁻ recq4a*^{+/-}*,* and then *zyp1-1*^{+/-} *recq4a*^{+/-} plants were crossed with *recq4b*^{+/-} plants. The obtained triple heterozygous *zyp1-1*^{+/-} *recq4a*^{+/-} *recq4b*^{+/-} in Col were crossed with *zyp1-6*^{+/-} *recq4a*^{+/-} in Ler to generate *zyp1-1*/*zyp1-6 recq4a*^{-/-} *recq4b*^{-/-} triple homozygous mutant in Col/Ler hybrid background; *recq4a*^{+/-} *recq4b*^{+/-} in Col were crossed with *recq4a*^{+/-} in Ler to generate *recq4a*^{*-*/-} *recq4b*^{*-*/-} double mutant in Col/Ler hybrid background; *zyp1-1*^{+/-} were crossed with *zyp1-6*^{+/-} plants to get *zyp1-1*/*zyp1-6* mutant in Col/Ler hybrid background, wild type controls were sister plants from these populations. *zyp1-1*/*zyp1-6 recq4a*^{*-*/-} *recq4b*^{-/-}*, recq4a*^{-*l*-} *recq4b*^{-/-}*, zyp1-1*/*zyp1-6* and wild type plants were backcrossed with Col male and female plants, respectively, to get the BC1 population for sequencing and subsequent CO analysis. BC1 populations were grown in the green house for three weeks (16-h day/ 8-h night) and four days in the dark. For DNA extraction and library preparation, 100-150 mg leaf samples were collected from the four BC1 plantlets¹².

### CO identification and analysis

In this study, the female and male population of wild type (95 and 89 plants), *zyp1* (0 and 42 plants), *HEI10OE* (48 and 46 plants), *recq4ab* (117 and 116 plants), *recq4ab HEI10OE* (143 and 139 plants) and *zyp1 recq4ab* (143 and 141 plants) were sequenced by Illumina HiSeq3000 (2×150bp) conducted by the Max Planck-Genome-center (https://mpgc.mpipz.mpg.de/home/). The raw sequencing data of the female and male population of wild type (428 and 294 plants, respectively, ArrayExpress number: E-MTAB-11254¹³), *zyp1* (272 and 225 plants, ArrayExpress number: E-MTAB-9593¹⁰, E-MTAB-11696¹⁴, *HEI10OE* (144 and 141 plants, ArrayExpress number: E-MTAB-11696), and *zyp1 HEI10OE* (142 and 138 plants, ArrayExpress number: E-MTAB-11696) from previous study were also included in this study. In total, 525 and 383 wild type female and male, 272 and 267 *zyp1* female and male, 192 and 187 *HEI10OE* female and male, 117 and 116 *recq4ab* female and male, 142 and 138 *zyp1 HEI10OE* female and male, 143 and 139 *zyp1 recq4ab* female and male, 143 and 141 *zyp1 recq4ab* female and male plants were analyzed, separately. The raw sequencing data were quality-controlled using FastQC v0.11.9 (http://www.bioinformatics.babraham.ac.uk/projects/fastqc/). The sequencing reads were aligned to the *Arabidopsis thaliana* Col-0 TAIR10 reference genome, which was downloaded from TAIR^{15,16}, using BWA v0.7.15-r1140¹⁷, with default parameters. A set of Sambamba v0.6.8¹⁸ commands was used for sorting and removing duplicated mapped reads. The creation of the high-confidence SNP marker list between Col and Ler, meiotic CO detection (a sliding window-based method, with a window size of 50 kb and a step size of 25 kb), check and filtering of low covered and potential contaminated samples were performed according to previous protocols^{10,13,19-21}. Samples of each population were randomly selected for checking predicted COs manually by inGAP-family²⁰.

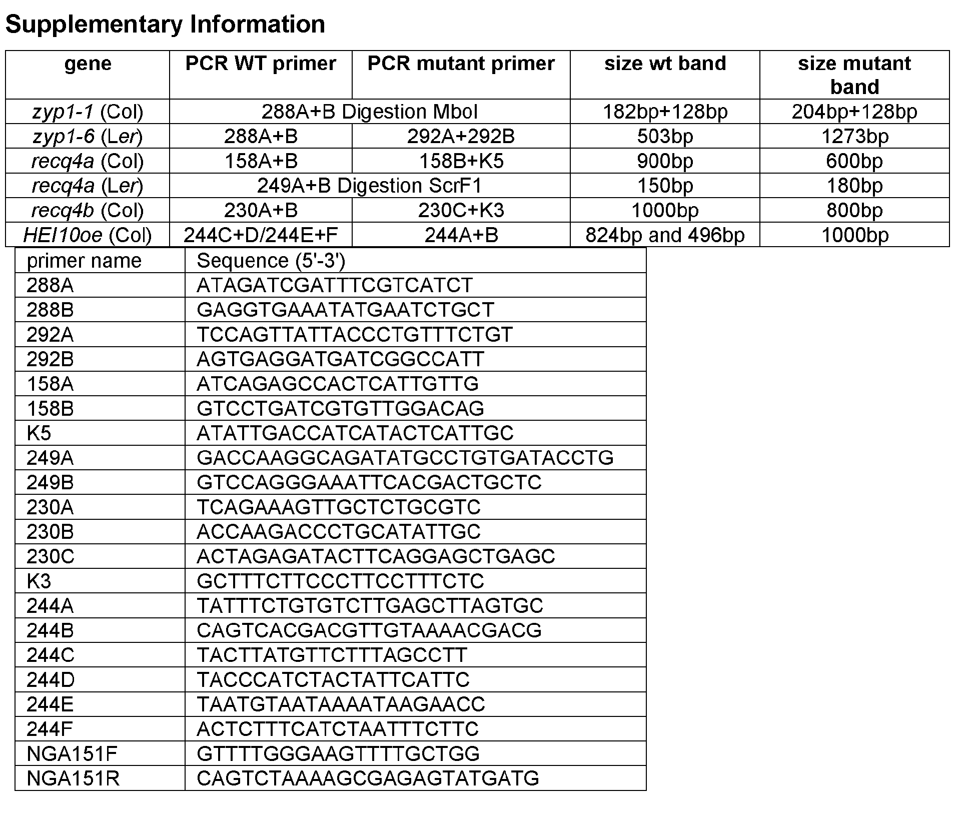

### References

1 Lian, Q. et al. The megabase-scale crossover landscape is largely independent of sequence divergence. Nat Commun 13, 3828, doi:10.1038/s41467-022-31509-8 (2022).
2 Capilla-Perez, L. et al. The synaptonemal complex imposes crossover interference and heterochiasmy in Arabidopsis. Proc Natl Acad Sci U S A 118, doi:10.1073/pnas.2023613118 (2021).
3 Giraut, L. et al. Genome-wide crossover distribution in Arabidopsis thaliana meiosis reveals sex-specific patterns along chromosomes. PLoS Genet 7, e1002354, doi:10.1371/journal.pgen.1002354 (2011).
4 Serra, H. et al. Massive crossover elevation via combination of HEI10 and recq4a recq4b during Arabidopsis meiosis. Proceedings of the National Academy of Sciences 115, 2437-2442, doi:10.1073/pnas.1713071115 (2018).
5 Ziolkowski, P. A. et al. Natural variation and dosage of the HEI10 meiotic E3 ligase control Arabidopsis crossover recombination. Genes & Development 31, 306-317, doi:10.1101/gad.295501.116 (2017).
6 Durand, S. et al. Joint control of meiotic crossover patterning by the synaptonemal complex and HEI10 dosage. Nature communications 13, 5999, doi:10.1038/s41467-022-33472-w (2022).
7 Fernandes, J. B., Seguela-Arnaud, M., Larcheveque, C., Lloyd, A. H. & Mercier, R. Unleashing meiotic crossovers in hybrid plants. Proc Natl Acad Sci U S A 115, 2431-2436, doi:10.1073/pnas.1713078114 (2018).
8 Serra, H. et al. Massive crossover elevation via combination of HEI10 and recq4a recq4b during Arabidopsis meiosis. Proc Natl Acad Sci USA 115, 2437-2442, doi:10.1073/pnas.1713071115 (2018).
9 Ziolkowski, P. A. et al. Natural variation and dosage of the HEI10 meiotic E3 ligase control Arabidopsis crossover recombination. Genes Dev 31, 306-317, doi:10.1101/gad.295501.116 (2017).
10 Capilla-Pérez, L. et al. The synaptonemal complex imposes crossover interference and heterochiasmy in Arabidopsis. Proceedings of the National Academy of Sciences 118, e2023613118, doi:10.1073/pnas.2023613118 (2021).
11 Seguela-Arnaud, M. et al. Multiple mechanisms limit meiotic crossovers: TOP3aα and two BLM homologs antagonize crossovers in parallel to FANCM. Proceedings of the National Academy of Sciences 112, 4713-4718, doi:10.1073/pnas.1423107112 (2015).
12 Rowan, B. A., Patel, V., Weigel, D. & Schneeberger, K. Rapid and Inexpensive Whole-Genome Genotyping-by-Sequencing for Crossover Localization and Fine-Scale Genetic Mapping. G3 Genes/Genomes/Genetics 5, 385-398, doi:10.1534/g3.114.016501 (2015).
13 Lian, Q. et al. The megabase-scale crossover landscape is largely independent of sequence divergence. Nature communications 13, 3828, doi:10.1038/s41467-022-31509-8 (2022).
14 Durand, S. et al. Joint control of meiotic crossover patterning by the synaptonemal complex and HEI10 dosage. Nat Commun 13, 5999, doi:10.1038/s41467-022-33472-w (2022).
15 Lamesch, P. et al. The Arabidopsis Information Resource (TAIR): improved gene annotation and new tools. Nucleic Acids Research 40, D1202-D1210, doi:10.1093/nar/gkrl090 (2012).
16 The Arabidopsis Genome, I. Analysis of the genome sequence of the flowering plant Arabidopsis thaliana. Nature 408, 796-815, doi: 10.1038/35048692 (2000).
17 Li, H. & Durbin, R. Fast and accurate short read alignment with Burrows-Wheeler transform. Bioinformatics 25, 1754-1760, doi:10.1093/bioinformatics/btp324 (2009).
18 Tarasov, A., Vilella, A. J., Cuppen, E., Nijman, I. J. & Prins, P. Sambamba: fast processing of NGS alignment formats. Bioinformatics 31, 2032-2034, doi:10.1093/bioinformatics/btv098 (2015).
19 Qi, J., Chen, Y., Copenhaver Gregory, P. & Ma, H. Detection of genomic variations and DNA polymorphisms and impact on analysis of meiotic recombination and genetic mapping. Proceedings of the National Academy of Sciences 111, 10007-10012, doi:10.1073/pnas. 1321897111 (2014).
20 Lian, Q., Chen, Y., Chang, F., Fu, Y. & Qi, J. inGAP-family: Accurate Detection of Meiotic Recombination Loci and Causal Mutations by Filtering Out Artificial Variants due to Genome Complexities. Genomics, Proteomics & Bioinformatics 20, 524-535, doi:https://doi.org/10.1016/j.gpb.2019.11.014 (2022).
21 Wang, H. et al. The cohesin loader SCC2 contains a PHD finger that is required for meiosis in land plants. PLOS Genetics 16, e1008849, doi:10.1371/journal.pgen.1008849 (2020).

## Claims

1. A method for increasing the frequency of meiotic crossover in a plant, plant part, plant tissue culture or plant cell comprising inhibiting in said plant, plant part, plant tissue culture or plant cell the expression and/or the function of the proteins ZYP1 and RECQ4.

2. A method for preparing a plant, plant part, plant tissue culture or plant cell displaying increased frequency of meiotic crossover in plants, comprising
(a) inhibiting in said plant, plant part, plant tissue culture or plant cell the expression and/or the function of the proteins ZYP1 and RECQ4, and
(b) optionally propagating the plant, plant part, plant tissue culture or plant cell.

3. The method of claim 1 or 2, wherein the inhibition of the proteins ZYP1 and RECQ4 is obtained by mutagenesis of the genes encoding said proteins or their promoter.

4. The method of claim 3, wherein the mutagenesis is the knock-out of the genes encoding ZYP1 and RECQ4 in the plant, plant part, plant tissue culture or plant cell..

5. The method of claim 4, wherein the knock-out is a homozygous knock-out of the genes encoding ZYP1 and RECQ4.

6. The method of any one of claims 3 to 5, wherein the mutagenesis is obtained by CRISPR-Cas technology, a meganuclease technology, a zinc finger nuclease technology, transcription activator-like (TAL) effector (TALE) nuclease technology or targeting induced local lesions (TILLING) technology.

7. The method of claim 1 or 2, wherein the inhibition of the proteins ZYP1and RECQ4 is obtained by one or more inhibitors of the expression of the proteins ZYP1 and/or RECQ4, and/or by one or more inhibitors of the function of the proteins ZYP1 and/or RECQ4.

8. The method of claim 7, wherein the one or more inhibitors of the expression of the proteins ZYP1 and/or RECQ4 are each independently selected from a small molecule, an aptamer, a siRNA, a shRNA, a miRNA, a ribozyme, and an antisense nucleic acid molecule.

9. The method of claim 7, wherein the one or more inhibitors of the function of the proteins ZYP1 and/or RECQ4 are selected from a small molecule, an antibody, an aptamer and an antibody mimetic, wherein the antibody mimetic is preferably selected from affibodies, adnectins, anticalins, DARPins, avimers, nanofitins, affilins, Kunitz domain peptides, Fynomers, trispecific binding molecules, evibodies, and probodies.

10. A plant, plant part, plant tissue culture or plant cell, wherein the expression and/or the function of the proteins ZYP1 and RECQ4 is inhibited.

11. The plant, plant part, plant tissue culture or plant cell of claim 10, wherein the genes encoding ZYP1 and RECQ4 are mutated, are preferably knocked-out and are most preferably homozygously knocked-out.

12. The method, plant, plant part, plant tissue culture or plant cell of any preceding claim, wherein the ZYP1 protein
(a) comprises or consists of an amino acid sequence being with increasing preference at least 50%, at least 60%, at least 70%, at least 80%, at least 90%, and at least 95% to SEQ ID NO: 1 or is encoded by a nucleotide sequence being with increasing preference at least 50%, at least 60%, at least 70%, at least 80%, at least 90%, and at least 95% to SEQ ID NO: 2; and/or
(b) comprises or consists of an amino acid sequence being with increasing preference at least 50%, at least 60%, at least 70%, at least 80%, at least 90%, and at least 95% to SEQ ID NO: 3 or is encoded by a nucleotide sequence being with increasing preference at least 50%, at least 60%, at least 70%, at least 80%, at least 90%, and at least 95% to SEQ ID NO: 4; and/or
the RECQ4 protein
(c) comprises or consists of an amino acid sequence being with increasing preference at least 50%, at least 60%, at least 70%, at least 80%, at least 90%, and at least 95% to SEQ ID NO: 5 or is encoded by a nucleotide sequence being with increasing preference at least 50%, at least 60%, at least 70%, at least 80%, at least 90%, and at least 95% to SEQ ID NO: 6, and/or
(d) comprises or consists of an amino acid sequence being with increasing preference at least 50%, at least 60%, at least 70%, at least 80%, at least 90%, and at least 95% to SEQ ID NO: 7 or is encoded by a nucleotide sequence being with increasing preference at least 50%, at least 60%, at least 70%, at least 80%, at least 90%, and at least 95% to SEQ ID NO: 8.

13. The method, plant, plant part, plant tissue culture or plant cell of any preceding claim, wherein the plant is or the plant part, plant tissue culture or plant cell is derived from a plant being selected from *Arabidopsis* (e.g. *A*. *thaliana*)*,* potato, cucumber, watermelon, sweet potato, eggplant, lettuce, cow pea, cassava., peanut, cotton, chick pea, onion, apple, corn, rice, wheat, barley, sorghum, millet oats, rye, triticale, cereals, soybean, alfalfa, leguminous crops, sugar beet, fodder beet, papaya, banana, plantains, fruits, grapevines, nuts, oilseed rape, sunflower, oil crops, squash cucumber, melons, cucurbits, cotton, fiber plants, sugarcane, palm, jatropha, fuel crops, cabbages, tomato, pepper, vegetables, ornamentals, shrubs, poplar, eucalyptus, trees, evergreens, grasses, coffee plants, tea plants, tobacco plants, hop plants, and rubber.

14. The method, plant, plant part, plant tissue culture or plant cell of any preceding claim, wherein
(a) the plant part is selected from seeds, leaves, flowers, anthers, ovules, fruits, stem cultures, rhizomes, tubers and bulbs, and is preferably flowers anthers, or ovules;
(b) the plant tissue culture is selected from flower tissue, anthers tissue, ovules tissue, meristematic tissue, protective tissue, parenchyma, sclerenchyma, collenchyma, xylem and phloem, and is preferably flower tissue, anthers tissue or ovules tissue; and/or
(c) the plant cell is from callus tissue, plant embryos, meristematic regions, reproductive tissues, meiocytes, gametophytes, sporophytes, pollen, egg, macrospore, or microspores and is preferably from meiocytes.
